# EUROPEAN PATENT APPLICATION

(11) **EP 3 744 273 A1**
(43) Date of publication of application: **02.12.2020**
(21) Application number: 20382401.6
(22) Date of filing: 13.05.2020
(51) Int. Cl.: A61B 17/70, A61B 17/86

(54) **INTERVERTEBRAL STABILISING SCREW**

(30) Priority: 21.05.2019 ES 201930856 U
(71) Applicant: Endospine, S.L., AD100 Saint Pere del Tarter (Parroquia de Canillo) (AD)
(72) Inventor: MORGENSTERN LOPEZ, Rudolf, 08950 Esplugues de Llobregat (Barcelona) (ES); MORGENSTERN DE MULLER, Christian Rudolf, 08950 Esplugues de Llobregat (Barcelona) (ES)
(74) Representative: Durán-Corretjer, S.L.P.

(57) **Abstract**

An intervertebral stabilisation screw that comprises a main body with a distal bone - fixation thread, said distal thread being located at a distal end of the main body, and a receiving area for a percutaneous drive tool at a proximal end, in which the screw also has externally and separated from the distal thread a proximal bone fixation thread located at a proximal end of the screw.

## Description

The present invention relates to an intervertebral stabilisation screw that can be used for intervertebral fusions.

Intervertebral stabilisation screws are known that are intended for being installed passing through the facet of a vertebra and entering the pedicle of the lower vertebra without affecting the vertebral ring. These vertebral stabilisation screws comprise a distal thread intended for being screwed into the vertebra, in the pedicle area. They also comprise an axial through-opening that is used for guiding the screw. The screws can also comprise a proximal portion capable of moving relative to the main body, whose function is to adjust the operational length of the screw.

One aim of the present invention is to disclose an intervertebral stabilisation screw that can be used for performing an improved, straightforward and quick disc treatment. This screw is suitable for outpatient surgery. The screw disclosed in this invention is compact and has better consistency that the screws commonly used for intervertebral stabilisation, and can be used for vertebral distraction (increase of the separation between vertebrae).

The screw that is the subject matter of the present invention comprises two threads, each intended for being screwed into respective contiguous vertebrae. In accordance with the present invention, the preferred installation of the screw can be carried out via the transpedicular route.

One aim of the present invention is to disclose an intervertebral stabilisation screw that comprises a main body with a distal bone fixation thread, the distal thread being located at a distal end of the main body, and a receiving area for a percutaneous drive tool at a proximal end, with the peculiarity that the screw also has externally and separated from the distal thread a proximal bone fixation thread located at a proximal end of the screw.

The main body preferably comprises an axial through-opening. Alternatively, the screw is solid. In one embodiment, the screw comprises a single part and, optionally, a cap.

Preferably, the thread direction of the proximal screw thread is the same as the thread direction of the distal screw thread, although it could be opposite. More preferably, the outer diameter of the proximal thread is larger than the diameter of the distal thread.

Preferably, the main body has at least one filling opening for bone remodelling composition, or a natural or artificial bone grafting material, in a side wall between the distal thread and the proximal thread. More preferably, the said filling opening(s) follow(s) a radial path. The said filling opening(s) preferably follow(s) a diametral path, with two outlets that connect opposing points of the wall of the main body.

In another preferred embodiment, the proximal thread is located in a hollow proximal secondary body independent from the main body. The main body and the secondary body are preferably screwed together. More preferably, the main body and the secondary body are screwed together via an outer thread of the main body and an inner thread of the secondary body. In this embodiment, in a more preferably configuration, the thread direction of the said outer thread of the main body and the inner thread of the secondary body is opposed to the thread direction defined by the distal and proximal threads.

Even more preferably, the secondary body has at its proximal end devices for receiving a percutaneous tool.

The screw preferably comprises a proximal cap for closing the access to the axial opening once the screw is installed.

A series of drawings of one embodiment of the present invention are appended to ensure better understanding through explanatory but non-exhaustive examples.
Figure 1 shows an exploded perspective view of one embodiment of a screw according to the present invention.
Figure 2 shows a diametral cross-section view of the screw of Figure 1.
Figure 3 shows an exploded perspective view of a second embodiment of a screw according to the present invention.
Figure 4 shows a diametral cross-section view of the screw of Figure 3.
Figure 5 shows a diametral cross-section view of a third embodiment of the screw.
Figure 6 shows, from a side viewpoint, a screw that is the subject of the present invention already placed between two vertebrae of a vertebral column.

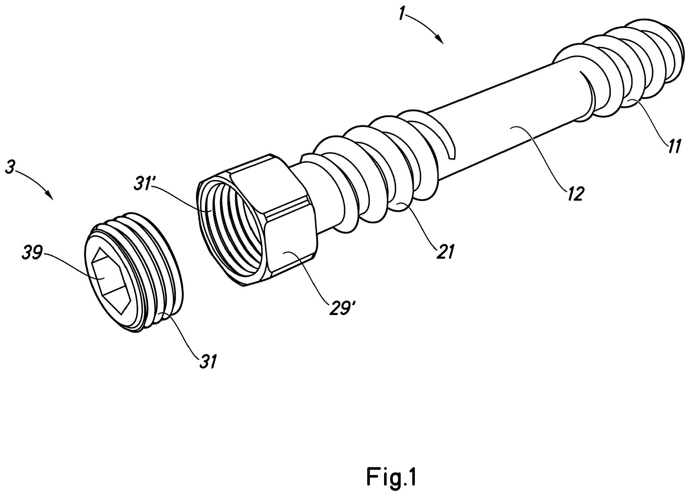

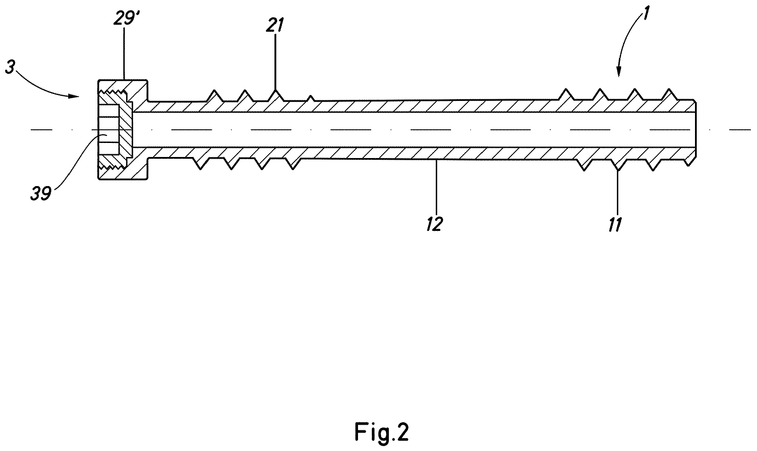

Figures 1 and 2 show a first embodiment of an intervertebral stabilisation screw that is the subject of the present invention.

Figure 1 shows an example of a screw that comprises a main body 1 with a distal thread 11 and a proximal bone fixation thread 21 located on the outside and separated from the distal thread 11 by an area 12. In the example of the figure, the distal thread 11 is located at a distal end of the main body 1, and the proximal thread 21 is located at a proximal end of the main body 1. Each of the said threads 11, 21 is intended for being screwed into respective contiguous vertebrae.

In this embodiment, both threads are located on a single main body 1.

Since the proximal thread 21 must be fixed to the bone in an area through which the distal thread 11 has already passed, the outer diameter of the proximal thread 21 is preferably larger than the diameter of the distal thread 11 in order to improve fixation.

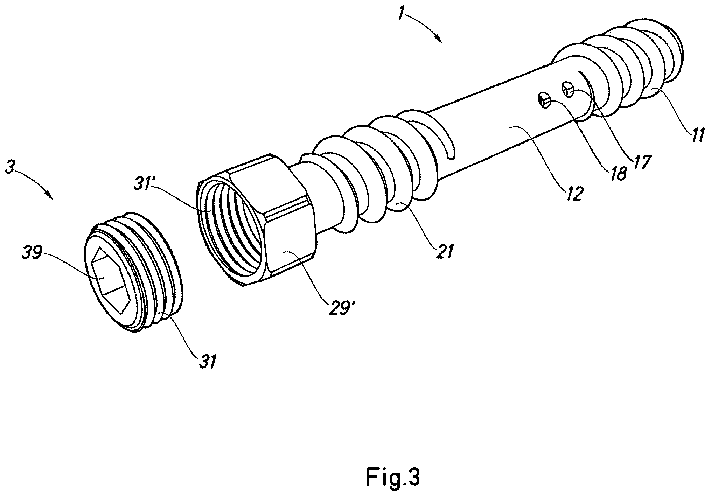

The thread direction of both threads is preferably the same, as can be observed in figure 1 and 3.

The thread direction of the proximal screw thread 21, when being a single main body 1, is generally the same to the thread direction of the distal screw thread 11, facilitating its insertion.

In the example of Figures 1 and 2, the proximal end 29' of the main body 1 comprises an outer hexahedral portion for receiving a percutaneous drive tool and an inner thread 31' for receiving a proximal cap 3 that closes the access to the axial opening once the screw has been installed. The cap 3 has a thread 31 mating with the inner thread 31' of the main body 1 and a hexahedral recess 39 for receiving an actuating tool.

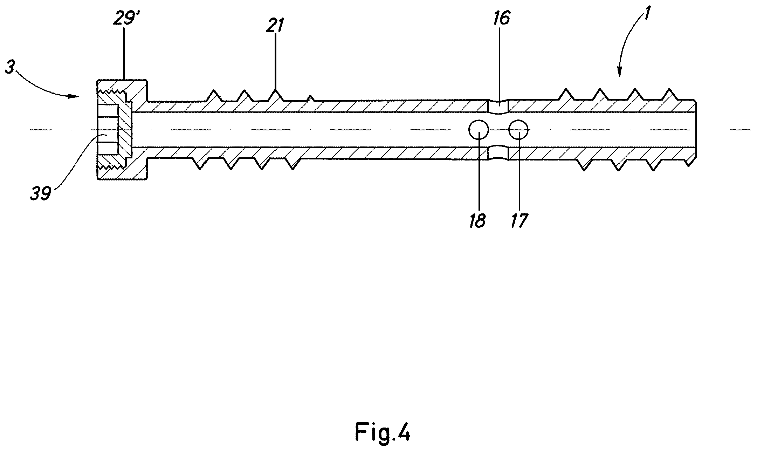

Figures 3 and 4 show a second embodiment of an intervertebral stabilisation screw. In said figures, elements that are the same or equivalent to those shown in the previous figures have been identified with the same numbers and, therefore, will not be explained in detail. In the example, the main body 1 has in a side wall, specifically in the area 12 between the distal thread 11 and the proximal thread 21, at least one filling opening 16, 17, 18 for connecting an intervertebral space with the axial opening of the main body 1. Specifically, the shown screw has three filling openings 16, 17, 18 (see Figure 4).

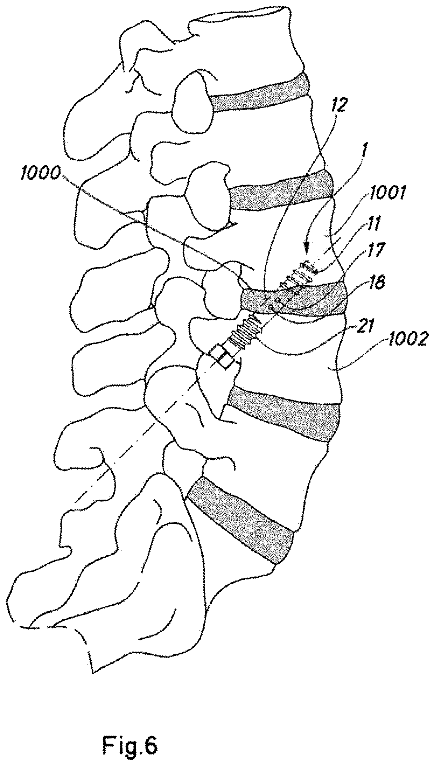

These filling openings 16, 17, 18 follow a diametral path, with two outlets that connect opposing points of the wall of the main body 1. The arrangement allows the surgeon who is performing the operation to check that there is at least one opening in the intervertebral space. The filling openings 16, 17, 18 are provided for injecting a bone remodelling composition, or a natural or artificial bone grafting material. The inside of the vertebral disc or intervertebral space 1000 (see Figure 6) is filled with said composition via the filling openings 16, 17, 18 and the axial opening. The arrangement of the filling openings 16, 17, 18 can be symmetric or asymmetric.

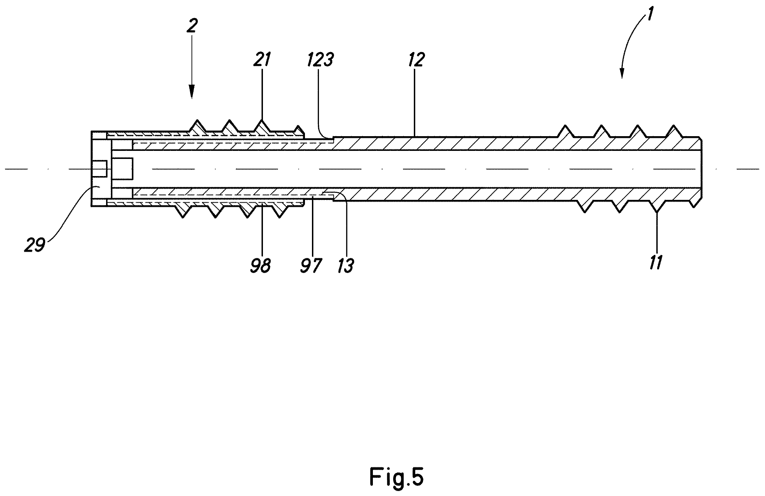

Figure 5 shows a third embodiment of a screw that is the subject of the present invention. In this figure, elements that are the same or equivalent to those shown in the previous figures have been identified with the same numbers and, therefore, will not be explained in detail.

The example of a screw shown in Figure 5 comprises two parts that are movable relative to one another: a main body 1 and a proximal secondary body 2. The main body 1 has at its distal end a distal bone fixation thread 11, while the secondary body 2 has a proximal bone fixation thread 21. Unlike the previous embodiments, the proximal thread 21 is located in a proximal secondary body 2 independent from the main body 1.One of the advantages of the embodiment with two bodies is that it makes it possible to modify the effective length of the screw and the distance between threads 11, 21, allowing vertebral distraction (increase of the separation between vertebrae).

In figure 5 the direction of the proximal thread 21 of the screw 2 and the thread direction of the distal thread 11 of the screw 1 are preferably the same. This allows to thread simultaneously the screw composed by 1 and 2 into the superior vertebral body and into the inferior vertebral pedicle. In a more preferably embodiment of this invention, the thread direction of the threads 97 and 98 is opposite to the one in the threads 11 and 21. This means that when the component 1 rotates on the component 2 in the thread direction of the thread 11, the body 1 is loosened from the body 2, leading to a lengthening of the screw that facilitates the vertebral distraction.

The main body 1 has two areas 12, 13 separated by an abutment 123 formed by a step generated by a variation of the outer diameter of the main body 1. This abutment is optional, therefore it might not exist, or it might adopt a very different shape and position. In this way, the proximal secondary body 2, which has a proximal bone fixation thread 21 can slide along the most proximal area 13 but not over the distal area 12, since it has a larger outer diameter.

In the example of Figure 5, both the main body 1 and the secondary body 2 comprise an axial opening, and thus are hollow. The travel limit of the proximal portion 2 is defined by the interference of the most distal face thereof with the abutment 123. The abutment 123 can have different shapes.

In addition, the axial opening of the main body 1 ends in a distal opening. This makes it possible to guide the travel of the main body 1. Alternatively, the main body 1 can be solid.

In a similar manner to the embodiment of figures 1, 2, 3 and 4, the thread direction of the proximal thread 21 of the main body 1 is the same as the one of the distal thread 11 of the secondary body 2, although it could be different, and the outer diameter of the proximal thread 21 is the same as the diameter of the distal thread 11, although preferably it could be larger.

In Figure 5, the main body 1 and the secondary body 2 are screwed together. The main body 1 and the secondary body 2 are screwed together via respective mated threads 97, 98. These mated threads comprise an outer thread 97 of the main body 1 and an inner thread 98 of the secondary body 2. This causes the movement between the two bodies to be also associated with a rotation between the two. To accomplish this, it is preferable that the distal and proximal threads share the same thread direction, this being opposite to the thread direction of the mated threads, although in an alternative embodiment the thread direction of all threads could be the same. In another embodiment, the thread direction of the mated threads could be coincident with the thread direction of either the distal or proximal thread

In addition, the embodiment of Figure 5 also provides for both the main body 1 and the secondary body 2 to have at their proximal end 29 respective areas or devices for receiving a percutaneous tool. The screw can also comprise a proximal cap (not shown in Figure 5) for closing the access to the axial opening once the screw is installed.

Figure 6 shows an example of preferred placement of a screw according to the embodiment of Figures 3 and 4. Said placement can also be used for other embodiments of screws according to the present invention. It is common to require the use of two screws. The preferred insertion thereof is transpedicular, passing through the disc space 1000 so that the distal thread 11 is screwed into the upper vertebra 1001 and the proximal thread 21 into the lower vertebra 1002.

The preferred access point in the case of percutaneous placement is located in the pedicle, at the centre of the superior articular process and approximately 1 mm below the lower edge of the transverse process of the vertebra, varying in accordance with the specific anatomy and other factors.

The angle of insertion of the screw varies in accordance with the specific anatomy of the vertebra. Its placement is transcutaneous, bilateral and pedicular. The use of transpedicular approach screws makes it possible to preserve the integrity of the intervertebral disc and the seal of the disc ring during the surgical approach of the intradiscal space.

During this screwing, the main body 1 enters until the distal thread 11 is screwed into the upper vertebra 1001, while the area 12 between the distal thread 11 and the proximal thread 21, which comprises the openings 17, 18, is entirely or partially inside the intervertebral ring space 1000. The proximal thread 21 of the proximal secondary body 2 makes it possible to fix the screw to a vertebra adjacent to the vertebra that receives the distal thread. The filling opening makes it possible to fill the intervertebral space 1000 (intra-annular space) with a bone remodelling composition, or a natural or artificial bone grafting material.

Once the screws are in place, it is possible to proceed, among others, with filling the space generated in the disc space with a bone remodelling composition, for which purpose any type of polymerisable bone cement can be used.

The installation process may be different from that described, and different percutaneous techniques and even non-percutaneous techniques may be used. The order of the operations is also subject to change.

Although the invention has been described in relation to preferred embodiments, these should not be understood to have any limiting effect on the invention, which will be defined by the broadest interpretation of the following claims.

## Claims

1. An intervertebral stabilisation screw that comprises a main body with a distal bone fixation thread, said distal thread being located at a distal end of the main body, and a receiving area for a percutaneous drive tool at a proximal end, **characterised in that** the screw also has externally and separated from the distal thread a proximal bone fixation thread located at a proximal end of the screw.

2. The screw according to the preceding claim, **characterised in that** the main body comprises an axial through-opening.

3. The screw according to any preceding claim, **characterised in that** the outer diameter of the proximal thread is larger than the diameter of the distal thread.

4. The screw according to any preceding claim, **characterised in that** the main body has at least one filling opening in a side wall between the distal thread and the proximal thread.

5. The screw according to any preceding claim, **characterised in that** it comprises at least two filling openings.

6. The screw according to either claim 4 or claim 5, **characterised in that** the said filling opening(s) follow(s) a radial path.

7. The screw according to any one of claims 4 to 6, **characterised in that** the said filling opening(s) follow(s) a diametral path, with two outlets that connect opposing points of the wall of the main body.

8. The screw according to any preceding claim, **characterised in that** it is formed by a single part.

9. The screw according to any one of claims 1 to 8, **characterised in that** the proximal thread is located in a hollow proximal secondary body independent of the main body.

10. The screw according to claim 9, **characterised in that** the main body and the secondary body are screwed together.

11. The screw according to claim 10, **characterised in that** the main body and the secondary body are screwed together via an outer thread of the main body and an inner thread of the secondary body.

12. The screw according to claim 11, **characterised in that** the thread direction of the said outer thread of the main body and the inner thread of the secondary body is opposed to the thread direction defined by the distal and proximal threads.

13. The screw according to any one of claims 9 to 12, **characterised in that** the secondary body has at its proximal end devices for receiving a percutaneous tool.

14. The screw according to any preceding claim, **characterised in that** it comprises a proximal cap for closing the access to the axial opening once the screw is installed.
